# EUROPEAN PATENT SPECIFICATION

(11) **EP 1 458 883 B1**
(45) Date of publication and mention of the grant of the patent: **16.05.2007**
(21) Application number: 02804896.5
(22) Date of filing: 13.12.2002
(51) Int. Cl.: C12Q 1/37

(54) **SCREENING METHOD FOR AGENTS USEFUL IN TREATING DIABETES**
SCREENING-VERFAHREN FÜR SUBSTANZEN ZUR BEHANDLUNG VON DIABETES
PROCEDE DE CRIBLAGE D'AGENTS UTILISES DANS LE TRAITEMENT DU DIABETE

(30) Priority: 14.12.2001 GB 0130007
(43) Date of publication of application: 22.09.2004
(73) Proprietor: Novartis Forschungsstiftung, Zweigniederlassung Friedrich Miescher Institute for Biomedical Research, 4058 Basel (CH)
(72) Inventor: HANASHIRO, Kazuhiko, Okinawa-city Okinawa 904-2161 (JP); NAGAMINE, Yoshikuni, CH-4125 Riehen (CH)
(86) International application number: PCT/EP2002/014233
(87) International publication number: WO 2003/052124

(56) References cited:
- WO-A-01/74793
- HELIN K ET AL: "A CDNA ENCODING A PRB-BINDING PROTEIN WITH PROPERTIES OF THE TRANSCRIPTION FACTOR E2F" CELL, CELL PRESS, CAMBRIDGE, NA, US, vol. 70, no. 2, 24 July 1992 (1992-07-24), pages 337-350, XP000872846 ISSN: 0092-8674
- LOSKUTOFF DAVID J ET AL: "The adipocyte and hemostatic balance in obesity: Studies of PAI-1." ARTERIOSCLEROSIS THROMBOSIS AND VASCULAR BIOLOGY, vol. 18, no. 1, January 1998 (1998-01), pages 1-6, XP002247362 ISSN: 1079-5642
- KOZICZAK MAGDALENA ET AL: "E2F1-mediated transcriptional inhibition of the plasminogen activator inhibitor type 1 gene." EUROPEAN JOURNAL OF BIOCHEMISTRY, vol. 268, no. 18, September 2001 (2001-09), pages 4969-4978, XP002247363 ISSN: 0014-2956
- SOBEL BURTON E ET AL: "Increased plasminogen activator type 1 in coronary artery atherectomy specimens from type 2 diabetic compared with nondiabetic patients: A potential factor predisposing to thrombosis and its persistence." CIRCULATION, vol. 97, no. 22, 9 June 1998 (1998-06-09), pages 2213-2221, XP002247364 ISSN: 0009-7322

## Description

The present invention relates to the fields of diabetes, obesity, pharmacology and medicine. More particularly, the invention relates to screening methods for identifying agents useful in treating diabetes.

Diabetes mellitus is characterized by the clinical syndrome of elevated blood glucose and is one of the leading causes of death in developed western countries. According to the responsiveness to insulin treatment, the disease is divided into two types: insulin-dependent (IDDM) and non-insulin-dependent (NIDDM), termed type 1 and type 2, respectively. They differ in the cause and course of disease, genetic susceptibility, incidence, and pathology. In most cases, IDDM occurs early in life and is usually associated with a genetic disorder leading to a defect in insulin production. Patients with IDDM respond to insulin, whereas NIDDM patients do not. NIDDM is far more prevalent than IDDM, NIDDM patients making up 85-90% of all diabetic patients. NIDDM is a rather late onset disease, the cause of which has not been determined, except that it has been linked to life style, showing a strong association with obesity. Insulin concentration is normal but blood sugar content is rather high and insulin administration has no influence despite the presence of insulin receptors on the cell surface of target cells. The absence of an insulin response seems to be due to impaired insulin utilization.

Plasminogen activator inhibitor type I (PAI-1) is a specific inhibitor of urokinase-type and tissue-type plasminogen activators, enzymes that play an important role in fibrinolysis (Vassalli, JD. et al., 1991, J Clin Invest., 88(4):1067-72; Loskutoff, D.J. et al., 1998, Arterioscler Thromb Vasc Biol., 18(1):1-6). Elevated plasma concentrations of PAI-1 detected in diabetic patients (Juhan-Vague, I. et al., 1996, Ann Med. 28:371-380) are thought to trigger deposition of fibrin clots, which lead to an increased risk of cardiovascular complications such as atherosclerosis (Dawson, S. et al., 1992, Atherosclerosis. 95:105-117; Juhan-Vague, I. et al., 1996, Ann Med. 28:371-380; Loskutoff, D.J. et al., 1998, Arterioscler Thromb Vasc Biol.,18(1):1-6; Sobel, B.E., et al., 1998 Circulation, 97(22):2213-21; Meigs, J.B., et al., 2000, JAMA, 283(2):221-8). Loskutoff, D.J. et al (supra) discloses that PAI-1 is elevated in obesity/NIDDM and appear to involve adipose tissue itself, as well as multiple cytokines, hormones, and growth factors. The PAI-1 mRNA profiles induced in the adipose tissue of insulin-, TNF-α-, and TGF-β-treated mice, when superimposed, appear to largely recapitulate the characteristic profile seen in the adipose tissue of obese mice. This fact, together with the observations that insulin, TNF-α, and TGF-β are elevated in obesity and induce PAI-1 in the plasma and adipose tissue of lean mice, lead the authors to speculate the involvement of these mediators in the regulation of PAI-1 in obesity. Sobel, B.E., et al., (supra) disclose that insulin can stimulate PAI-1 synthesis in vivo and that increased PAI-1 is potentially predisposing to thrombosis, acute occlusion, and accelerating atherosclerosis because of thrombus-associated mitogens, present in excess in atheroma from type 2 diabetic subjects. The increased production of PAI-1 seen in diabetic disease has been attributed directly to high glucose levels in the blood (Nordt, T. K. et al., 1993, Arterioscler Thromb. 13:1822-1828). Glucose regulates PAI-1 gene expression through two Spl sites located between - 85 and - 42 of the PAI-1 promoter in vascular smooth muscle cells (Chen, Y. Q. et al., 1998, J Biol Chem. 273:8225-8231).

PAI-1 expression has been observed in various cell types, and multiple regulatory factors have been identified that play a role in PAI-1 transcription, including growth factors (TGF-β, EGF, platelet-derived growth factor, BFGF), inflammatory cytokines (IL-1, TNF-α) and hormones (corticosteroids, insulin). Fat tissue is one of the major sources of PAI-1 in the body (Loskutoff, D.J. et al., 1998, Arterioscler Thromb Vasc Biol., 18(1):1-6,). In particular, visceral fat is the main source of PAI-1 in diabetic patients (Mertens, I., et al., 2001, Horm Metab Res., 33(10):602-7). Several protein factors including TNFα (Samad, F., 1996, J Clin Invest., 97(1):37-46), TGFβ (Alessi, M.C., 1997, Diabetes, 46(5):860-7), leptin (De Mitrio, V., et al., 1999, Metabolism, 48(8):960-4), angiotensin II (Skurk, T., et al., 2001, Hypertension, 37(5):1336-40) and insulin (Samad, F. et al., 1996, Mol Med, 2(5):568-82) have been proposed as mediators of high PAI-1 expression in adipocytes, the major component of fat tissue.

Recently, E2F1 has been shown to suppress the PAI-1 gene in various types of dividing cells in culture, including LLC-PKL pig-kidney epithelial cells, HEK293 human embryonic, kidney epithelial cells, MEF mouse embryonic fibroblasts, WI-38 human lung fibroblasts, and U20S and SAOS-2 human osteosarcoma cells, in a manner independent of pocket-binding proteins but requiring the DNA-binding domain of E2F (Koziczak, M., et al., 2000, Mol Cell Biol 20(6): 2014-22; Koziczak, M., et al., 2001, Eur J Biochem 268(18): 4969-78). In these studies, it has also been shown that the negative regulation of the PAI-1 gene by the transcription factor E2F was transcriptional. Although the underlying molecular mechanism has not been fully understood, the action of E2F on the PAI-1 promoter seems to be direct, because suppression of the PAI-1 gene by activated E2F was not affected by the protein synthesis inhibitor cycloheximide (Koziczak, M., et al., 2001, Eur J Biochem 268(18): 4969-78). The question remains whether PAI-1 gene expression can be modulated in cells that are no longer cycling, in conditions where E2F is inactive. Adipocytes are terminally differentiated cells and as such no longer divide. Altiok et al. (Altiok, S. et al., 1997, Genes Dev. 1997 Aug 1;11 (15):1987-98) reported that E2F activity is reduced when preadipocytes are differentiated to adipocytes by PPARγ (peroxisome proliferator-activated receptor gamma) activation through a mechanism involving the downregulation of the serine/threonine protein phosphatase PP2A.

E2F transcription factors play a pivotal role in cell cycle events at the boundary of the G0/G1 and S phases regulating various genes required for DNA replication and cell cycle progression (Helin, K., 1998, Curr Opin Genet Dev, 8(1):28-35. Review; (Nevins, J.R., 1998, Cell Growth Differ. 1998 Aug;9(8):585-93. Review). Active E2F is a heterodimer of an E2F family member (E2F1-6) and a DP family member (DP1 or DP2) (Wu, C. L. et al, 1995, Mol Cell Biol. 15:2536-2546). E2F activity is regulated by interacting with pocket binding proteins (pRB, p107 and p130).
Binding of a pocket protein to E2F suppresses its transactivation activity (Cobrinik, D. et al., 1993, Genes Dev. 7:2392-2404; and Qin, X. Q. et al., 1995, Mol Cell Biol. 15:742-755) or converts it to an active repressor (Helin, K. et al., 1993, Mol Cell Biol. 13:6501-6508; and , Zhang, H. S. et al., 1999, Cell. 97:53-61), which exerts its inhibitory effect partly by recruiting histone deacetylase (Brehm, A. et al., 1998, Nature. 391:597-601) or by interacting with general transcription factors (Weintraub, S. J. et al., 1995, Nature. 375:812-815). Phosphorylation of pocket binding proteins by cyclin-dependent kinases releases active E2F at the G0/G1 to S phase boundary, which become inactive by phosphorylation of DP subunit by another cyclin-dependent kinase at the end of the S phase. Accordingly, E2F activity is decreased when cells are terminally differentiated or reach senescence (Good, L., et al., 1996, J Cell Physiol. 1996 Sep;168(3):580-8; Gill, R. M., et al., 1998, Exp Cell Res 244(1): 157-70), when the genome does not need to replicate.

There remains a need for agents effective in treating diabetes or its onset and this invention meets those needs. A method is provided for the screening for agents effective against diabetes by assaying for PAI-1 activity in the presence of a candidate agent; and correlating a decrease in PAI-1 activity relative to when the candidate agent is absent with the presence of a potential agent effective against diabetes. Preferably, the candidate agent decreases PAI-1 expression.

In one embodiment, PAI-1 expression is decreased by elevating E2F activity and thus, a decrease in PAI-1 activity can be inferred by detecting an increase in E2F activity. Thus, in a further aspect of the invention, a method of screening for agents effective against diabetes is provided, the method comprising assaying for E2F activity in the presence of a candidate agent; and correlating an increase in E2F activity relative to when the candidate agent is absent with the presence of a potential agent effective against diabetes.

An increase in E2F activity can be mediated by disrupting the interaction between E2F and pRB, using a peptide, for example. Thus, in another aspect of the invention, a method of screening for agents effective against diabetes is provided, the method comprising: assaying for an association between E2F and pRB in the presence of a candidate agent; and correlating a loss in E2F-pRB association (e.g., release of active E2F from the complex) relative to when the candidate agent is absent with the presence of a potential agent effective against diabetes.

PAI-1 activity is thought to affect ανβ3 integrin activity (see Example 6). Therefore, in a further aspect of the invention, a method of screening for agents effective against diabetes is provided, comprising:
(a) assaying for αvβ3 activity in the presence of a candidate agent; and
(b) correlating an increase in αvβ3 activity relative to when said candidate agent is absent with the presence of a potential agent effective against diabetes.

The candidate agent can be a small organic product, natural product or a peptide, for example. Antibodies, such as an activating antibody effective in activating αvβ3 activity or an inhibitory antibody, for example inhibiting the interaction between pRB and E2F, are also useful. Inhibitory nucleic acid molecules can also be potentially effective agents.

Thus, the present specification also relates to agents identified by any one of the screening methods of the invention. Such agents may be used for further development (e.g., to improve specificity, cellular uptake or other desired characteristic) or used in the manufacture of a medicament or as a pharmaceutical, optionally together with other components or active ingredients.

Thus, the specification also provides a method of treating an individual with diabetes or a predisposition to diabetes, by administering to the individual a pharmaceutically effective amount of an agent effective in lowering PAI-1 expression, in an amount sufficient to decrease PAI-1 expression in the individual.

The invention also provides a method of treating an individual with diabetes or a predisposition to diabetes, by administering to the individual a pharmaceutically effective amount of an agent effective in increasing E2F activity. The agent can therefore be E2F itself or an active fragment thereof (e.g., a fragment capable of inhibiting PAI-1 expression).

Furthermore, the specification also relates to a method of treating an individual with diabetes or a predisposition to diabetes, by administering to the individual a pharmaceutically effective amount of an agent effective in increasing ανβ3 integrin activity. The agent can be ανβ3 integrin itself or an active fragment thereof (e.g., a fragment capable of inhibiting PAI-1 activity) or an agent that increases ανβ3 integrin activity in the individual, such as an activating antibody, as is exemplified in Example 6 below.

PAI-1, a potent inhibitor of plasminogen activators, is expressed in a variety of tissues although recently it has become evident that adipose tissue is its main source. The high fat tissue content of patients suffering from obesity and type 2 diabetes results in high levels of PAI-1 circulating in the blood, which in turn results in cardiovascular complications, such as thrombosis and artherosclerosis (Loskutoff, D.J. et al., 1998, Arterioscler Thromb Vasc Biol., 18(1):1-6; Juhan-Vague, I., et al., 1997, Thromb Haemost 78(1): 656-60). The present inventors propose that elevated PAI-1 expression is a cause of NIDDM and is not merely a consequence of NIDDM. The present invention therefore provides screening methods for agents that are effective against diabetes or in preventing the onset of diabetes based on inhibiting PAI-1 expression, in particular in adipocytes.

Thus, the present invention provides methods for identifying candidate agents that prevent, ameliorate or correct pathological development of NIDDM. In particular, a method of screening for agents effective against diabetes is provided comprising assaying for PAI-1 activity, preferably PAI-1 expression, in the presence of a candidate agent and correlating a decrease in PAI-1 activity relative to when said candidate agent is absent with the presence of a potential agent effective against diabetes. PAI-1 activity or expression can be detected by any method known in the art or yet to be developed. The Examples below illustrate the invention using Northern blotting techiniques. However, it will be apparent to one of ordinary skill in the art that alternative methods can be used, such as the use of the polymerase chain reaction, reverse transcription, antibodies specific for PAI-1 and the like. Alternatively, a construct can be designed to express a heterologous sequence under the control of PAI-1 regulatory sequences. The PAI-1 (or E2F) expression level can then be detected by following the presence of a protein marker expressed under the control of PAI-1. The protein marker can be encoded by a reporter gene operably linked to a PAI-1 gene or an operable fragment thereof (i.e. capable of driving expression of the protein marker with the same expression pattern as PAI-1). Protein markers include without limitation β-galactosidase, glucosidases, chloramphenicol acetyltransferase (CAT), glucoronidases, luciferase, peroxidases, phosphatases, oxidoreductases, dehydrogenases, transferases, isomerases, kinases, reductases, deaminases, catalases, urease, and fluorescent proteins (e.g. GFP, RFP, YFP). Alternatively, the protein product, including PAI-1 or E2F can be detected using antibodies specific for the protein of interest, as is well known in the art.

The present inventors have demonstrated for the first time that E2F protein is present in differentiated adipocytes and can be reactivated to regulate PAI-1 gene expression. The reversal of E2F inactivation suppresses PAI-1 induction by insulin. Thus, in a further aspect of the invention, a method is provided for screening for agents effective against diabetes, comprising assaying for E2F activity in the presence of a candidate agent and correlating an increase in E2F activity relative to when the candidate agent is absent with the presence of a potential agent effective against diabetes. The E2F activity can be detected by monitoring PAI-1 expression, a decrease in PAI-1 expression reflecting an increase in E2F activity. The method of assaying for E2F activity is, however, not so limited and it will be apparent to one of ordinary skill in the art that other methods can be used, such as a modification of the expression assays described above, using a marker gene expressed under the control of E2F regulatory sequences or an antibody specific for E2F. However, it is preferred to assay for E2F activity (e.g., transcriptional activity or DNA binding activity) rather than merely detecting E2F expression. Now that the present inventors have demonstrated that the PAI-1 gene can be regulated in adipocytes by E2F activity, E2F provides a new target for treatment of the symptoms associated with diabetes, such as cardiovascular disorders, as well as diabetes itself.

In a preferred embodiment of the present invention, the increase in E2F activity is mediated by disrupting the interaction between E2F and pRB (pocket binding protein), for example using a peptide, peptide mimetic, natural product, antibody or small organic molecule. Thus, also provided by the invention is a method of screening for agents effective against diabetes, the method comprising assaying for an association between E2F and pRB in the presence of a candidate agent; and correlating a loss in association relative to when the candidate agent is absent with the presence of a potential agent effective against diabetes. The Examples below illustrate this aspect of the invention using electromobility shift assays (or gel shift assays; see Examples 2 and 3) although it will be apparent to one of ordinary skill in the art that other techniques can be easily applied for the same purpose. For example, one or more of the protein entities may be labelled (optionally, differentially if more than one protein entity is labelled) and the release of the chosen labelled entity detected as is usual in high through put formats. Alternatively, both interacting moieties may be labelled and assayed under conditions where there is fluorescence quenching when both labels are in close proximity (i.e., both protein moieties are bound to each other) but a fluorescent signal is detected upon release of one of the protein moieties (loss of association). Thus, the proteins may be immobilised on a solid carrier like a microtiter plate or beads; or may bear one or more identifiable markers like biotin or a radioactive, fluorescent or chemiluminescent group.

The present inventors have shown in time course analyses of adipogenesis using NIH3T3-L1 preadipocytic cells that PAI-1 mRNA levels are increased when cells are differentiated into adipocytes and its induction by insulin was also enhanced (see Example 1). The protein levels of the cell cycle-regulating transcription factor E2F1, the main component of E2F, remained constant. This reflects the fact that in terminally differentiated cells E2F is inactive. Analysis of E2F DNA-binding activity of nuclear extracts by electromobility shift assays showed that DNA-protein complexes shifted to higher molecular weight forms indicating that E2F inactivation that normally accompanies terminal differentiation, is not brought about by downregulation of the E2F protein level but by its interaction with another factor, a pocket-binding protein, such as pRB (see Example 2).

In one embodiment of the present invention, a synthetic peptide corresponding to the pRB-binding region of E2F1 is used to disrupt the interaction between E2F and pRB and suppress the formation of the high molecular weight, DNA-binding complex revealed in electromobility shift assays. Conjugation of this peptide to a cell-penetrating peptide derived from HIV tat protein (Schwarze, S. R., et al., 1999, Science 285(5433): 1569-72) allows this interfering peptide to enter a cell where it can act. Treatment of adipocytes with the cell-penetrating, interfering peptide reduced PAI-1 mRNA levels at basal as well as insulin induced levels whereas a control cell-penetrating peptide showed no effect (see Example 4).

Although exemplified in the specification using adipocytes as terminally differentiated cells, the invention has applications in other cells, in particular other terminally differentiated cells, including without limitation smooth muscle cells or hepatocytes, wherein insulin induction of PAI-1 can also be repressed by a similar mechanism, or indeed Rb-E2F regulation of other genes may be desired. Nevertheless, the screening assays of the invention are not dependent on a particular cell and indeed can be carried out in vitro using component parts.

PAI-1 activity is thought to affect ανβ3 integrin activity (see Example 6). Therefore, in a further aspect of the invention, a method of screening for agents effective against diabetes is provided, comprising assaying for ανβ3 activity in the presence of a candidate agent; and correlating an increase in ανβ3 activity relative to when the candidate agent is absent with the presence of a potential agent effective against diabetes. Any assay for αvβ3 integrin activity can be used, including assays similar to those described above for PAI-1 or E2F. For example, αvβ3 integrin expression can be detected using an antibody specific for αvβ3 integrin or expression assays described above, using a marker gene expressed under the control of αvβ3 integrin regulatory sequences. However, it is preferred to assay for an effect on αvβ3 integrin activity (e.g., increased vitronectin binding) or by monitoring PAI-1 expression, a decrease in PAI-1 expression reflecting an increase in αvβ3 integrin activity, rather than merely detecting expression.

The present specification also relates to agents identified by any one of the screening methods of the invention and their use as a pharmaceutical or in the manufacture of a medicament. Such agents may be small organic molecules, antibodies (in particular humanized antibodies), inhibitory nucleic acids, such as anti-sense oligonucleotides or siRNA, specific for PAI-1. SiRNA technology can be routinely applied based on sequences specific for PAI-1, and targeted expression of siRNAs can be achieved using tissue-specific promoters, such as promoters specific for adipose tissue. Antibodies may be used to block protein interactions or activity or to enhance activity, as is well known in the art.

Thus, the specification relates to pharmaceutical preparations comprising an active agent of the invention. Therefore, the present specification provides methods for treating an individual with a disease, disorder or condition of interest (in particular diabetes) with an agent identified by the methods of the invention. The present inventors envision that it would be particularly beneficial to control PAI-1 induction at the time of insulin surge after eating. Furthermore, an agent effective against diabetes, that decreases PAI-1 expression (or increases E2F or αvβ3 integrin activity), might be used on a daily basis for the prevention of diabetes.

The active agent can be mixed with excipients that are pharmaceutically acceptable and compatible with the active agent and in amounts suitable for use in the therapeutic methods described herein. Suitable excipients are, for example, water, saline, dextrose, glycerol, ethanol or the like and combinations thereof, including vegetable oils, propylene glycol, polyethylene glycol and benzyl alcohol (for injection or liquid preparations); and petrolatum, vegetable oil, animal fat and polyethylene glycol (for externally applicable preparations). In addition, if desired, the composition can contain wetting or emulsifying agents, isotonic agent, dissolution promoting agents, stabilizers, colorants, antiseptic agents, soothing agents and the like additives (as usual auxiliary additives to pharmaceutical preparations), pH buffering agents and the like which enhance the effectiveness of the active ingredient.

The therapeutic compositions can include pharmaceutically acceptable salts of the components therein. Pharmaceutically acceptable salts include the acid addition salts (e.g. formed with free amino groups) that are formed with inorganic acids such as, for example, hydrochloric or phosphoric acids, or such organic acids as acetic, tartaric, mandelic and the like. Salts formed with the free carboxyl groups can also be derived from inorganic bases such as, for example, sodium, potassium, ammonium, calcium, or ferric hydroxides, and such organic bases as isopropylamine, trimethylamine, 2-ethylamino ethanol, histidine, procaine and the like.

As used herein, the terms "pharmaceutically acceptable", "physiologically tolerable" and grammatical variations thereof, as they refer to compositions, carriers, diluents and reagents, are used interchangeably and represent that the materials are capable of administration to or upon a subject, e.g., a mammal, without the production of undesirable physiological effects such as nausea, dizziness, gastric upset and the like.

Physiologically tolerable carriers are well known in the art. Exemplary of liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, polyethylene glycol and other solutes. Liquid compositions can also contain liquid phases in addition to and to the exclusion of water. Exemplary of such additional liquid phases are glycerine, vegetable oils such as cottonseed oil, and water-oil emulsions.

Pharmaceutical compositions containing agents effective against diabetes may be administered peridurally, orally, rectally, parenterally, intravaginally, intraperitoneally, topically (as by powders, ointments, drops or transdermal patch), bucally, or as an oral or nasal spray. By "pharmaceutically acceptable carrier" is meant a non-toxic solid, semisolid or liquid filler, diluent, encapsulating material or formulation auxiliary of any type (see above). The term "parenteral" as used herein refers to modes of administration that include intravenous, intramuscular, intraperitoneal, intrasternal, subcutaneous and intraarticular injection and infusion. Preferably, the agent is administered orally, through food intake.

Further included in this specification is a method of specifically decreasing PAI-1 activity with an effective amount of a compound, as well as a method of specifically increasing E2F activity with an effective amount of a compound. Such a method may be employed for medical as well as preventive purposes. For example, a method of treatment of a disease with an effective amount of a compound according to this invention that decreases PAI-1 (or increases E2F or αvβ3 integrin), is included in this specification. Examples of such diseases include without limitation obesity, type 2 diabetes, cardiovascular disease, thrombosis, artherosclerosis. Thus, in a preferred embodiment, the invention provides a method of treating an individual with diabetes or a predisposition to diabetes, the method comprising administering to the individual a pharmaceutically effective amount of an agent effective in lowering PAI-1 expression, in an amount sufficient to decrease PAI-1 expression in the individual. The agent can be E2F or an active fragment thereof but agents that increase E2F or ανβ3 integrin activity in the individual, such as peptides, antibodies or organic molecules are preferred. E2F activity can be increased by disrupting the association of pRB and E2F, such as using a peptide mimetic or inhibitory antibody. Alternatively, PAI-1 itself can be targeted, for example, using siRNA technology (EP1 144 623).

The specification also relates to a composition comprising an agent identified by the methods of the invention and a pharmaceutically appropriate carrier, such as described above, as well as pharmaceutical packs or kits comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. Such kits can optionally comprise equipment useful in administering the composition such as an inhaler, syringes and the like.

The invention is further described below, for the purpose of illustration only, in the following examples.

### Example 1: Differentiation of NIH3T3-L cells

NIH3T3-L1 preadipocytes (ATCC No. CL-173) were induced to differentiate into adipocytes by treating confluent cells with a mixture of insulin, dexamethasone and isobutylmethylxanthine followed by a successive change of different media (Student, A. K. et al., 1980, J Biol Chem. 255:4745-4750). In brief, NIH3T3-L1 preadipocytic cells (2×10⁵ cells) were grown in 60-mm plastic dishes with 4 ml Dulbecco's modified Eagle's medium (DMEM) supplemented with 10% fetal bovine serum (FBS). Two days after cells reached confluency, the medium was changed to DMEM containing 10% FBS, 10 µg/ml insulin, 1 µM dexamethason, and 0.5 mM isobutylmethylxanthine. Two days later, this medium was replaced with DMEM supplemented only with 10 µg/ml insulin. Subsequently, two days later this medium was withdrawn and replaced with DMEM containing 10% FBS, and thereafter cells were replenished with the same medium every two days.

Adipocyte differentiation was monitored by staining lipids with oil-red O. Briefly, cells were washed twice with phosphate buffered saline (PBS) and fixed with 3.7% formaldehyde solution for 1 h. After washing twice with PBS, cells were stained with oil-red O [60:40 (v/v) dilution in water of 0.5% stock solution (v/v, in isopropanol)] for 1 h. Cells were then washed twice with PBS and twice with water. Lipid-positive cells started to appear after the third day of induction and reached more than 90% by the eighth day. PPARγ (peroxisome proliferator-activated receptor gamma) mRNA, another marker of adipocyte differentiation, also started, to appear at the third day and reached a plateau by the 5th day. For RNA analysis, total RNA (12 µg), prepared with acid guanidinium thiocyanate-phenol-chloroform method, was resolved by agarose gel electrophoresis under denaturing conditions and transferred to a nylon membrane as described (Northern blotting) (Ziegler, A., et al., 1991, J Biol Chem 266(31): 21067-74). Equal RNA loading and transfer to nylon membranes were confirmed by staining rRNA with methylene blue (Herrin, D. L. and G. W. Schmidt, 1988, Biotechniques 6(3): 196-7, 199-200). The DNA inserts from the plasmid were labelled with {α-³²P}dATP using the random oligo-primed reaction (Feinberg, A. P. and B. Vogelstein, 1983, Anal.Biochem., 132: 6-13). Levels of specific RNA were measured using a Molecular Dynamic Phosphorlmager.

The human, rat, and mouse PAI-1 genes have been isolated earlier and their promoter sequences characterized (Bosma, P.J., et al, 1988, J Biol Chem. 263:9129-41; Bruzdzinski, C.J. et al., 1990, J Biol Chem. 265: 2078-85; Loskutoff, D.J., et al, 1987, Biochemistry. 26:3763-68; Prendergast, G.C. et al., 1990, Mol Cell Biol. 10:1265-69). Comparison of 5'-flanking regions revealed two highly conserved elements with > 80% identity in the proximal promoter (at - 90 to - 25) and in a distal sequence (at - 753 to - 512). The common features detected in the 5'-flanking regions of the PAI-1 genes of these three species are a consensus TATA box and sequences closely related to PEA3, API, CTF/NF-1, and Spl recognition sites (Descheemaeker, K.A., et al., 1992, J Biol Chem. 267:15086-91;Johnson, M.R., et al., 1992, J Biol Chem. 267: 12202-10; Riccio, A., et al., 1988, Nucleic Acids Res. 16: 2805-24).

PAI-1 mRNA levels were low when cells were confluent and started to increase upon adipocytic differentiation. When cells were treated with insulin at different stages of differentiation, PAI-1 mRNA was induced to a very low extent (before cells were confluent) or not at all (when cells were confluent) but induced markedly in terminally differentiated cells (day 4 and 8).

To examine the level of E2F-1 protein, cells in a 60-mm plastic dish were washed with PBS and collected with 400 µl lysing buffer [20 mM Tris-HCl (pH 7.4), 137 mM NaCl, 2mM EDTA (Ethylenediamine tetraacetic acid), 1% Triton-X100, 10% glycerol, 10 µg/ml aprotinin, 10 µg/ml leupeptin, and 10 mM DTT] (Dithiothreitol). Samples (20 µg protein) were fractionated by SDS-polyacrylamide gel electrophoresis, blotted only polyvinylidene difluoride membranes, and analyzed using polyclonal antibodies against E2F1, 2 and 3. An enhanced chemiluminescence detection method (ECL; Amersham) was employed, and the membrane was exposed to Kodak Biomax MR film. Examination of the level of E2F-1 protein revealed that it remained constant during the time course of experiment although cells stopped growing after reaching confluence, raising the possibility that it is E2F activity not the level of E2F protein that is downregulated during adipocytic terminal differentiation. In accordance with this, analysis of other E2F1 family members E2F2 and 3, which we have shown to be negative regulators of the PAI-1 promoter (Koziczak, M., et al., 2000, Mol Cell Biol 20(6): 2014-22), also showed no decrease when cells were differentiated; E2F2 protein levels increased and E2F3 remained constant like E2F1. Measurement of E2F DNA-binding activity by electromobility shift assays (see Example 2) using nuclear extracts from cells at different stages showed a differentiation-dependent decrease, demonstrating that the regulation of E2F is posttranscriptional, most likely posttranslational (see Example 4).

### Example 2: Changes in DNA-binding patterns of E2F

Changes in E2F DNA-binding patterns during adipogenesis were investigated. Nuclear extracts were prepared from NIH3T3-L1 cells at different times during induction of differentiation and their E2F DNA-binding activity was assessed by DNA gel shift assays using a radio active E2pro oligonucleotide corresponding to two tandem E2F binding sites in the adenovirus E2 promoter. Oligonucleotide probes were radiolabeled using E. coli polynucleotide kinase and {γ-32P}ATP. The oligonucleotides used for electromobility shift assays (EMSA) were as follows (only upper strands are given): E2pro (corresponding to the -72 to -32 region of the adenovirus E2 promoter; E2F binding site), 5'-ATCAGTTTTCGCGCTTAAATTT GAGAAAGGGCGCGAAACTAG-3' (SEQ ID NO:1); CycD1 (E2F binding site from the cyclin D1 promoter), 5'-AATTCGCTGCTCCCGGCGTTTGGCGCCCGCGCC-3' (SEQ ID NO:2); CycD1 m, 5'-AATTCGCTGCTCCCGTCGTTAGGTGTCCGCGCC-3' (SEQ ID NO:3, mutated nucleotides in bold italic). Nuclear extracts (4 µg) were first incubated at room temperature for 15 min in 20 µl of binding reaction consisting of 50 mM KCI, 20 mM HEPES [4-(2-Hydroxyethyl)-1-piperazineethanesulfonic acid] (pH 7.9), 0.2 mM EDTA, 8% glycerol, 1 µg salmon sperm DNA, 6 µg bovine serum albumin and 1 mM DTT together with or without penetrating peptide and antibodies, followed by further 15 min incubation after addition of 0.3 ng of radiolabeled oligonucleotide probes. Aliquots (5 µl) of reaction mixtures were separated in a 4.5% polyacrylamide gel run in 0.25xTBE (Tris-Borate-EDTA) buffer at room temperature. The gel was dried and analyzed using a Phosphorlmager.

The pattern of DNA-binding of nuclear proteins to the E2F sites of the Adenovirus E2 promoter as revealed by gel shift assays showed growth-associated changes. Before cells reached confluence, the DNA-protein complex was of lower molecular weight, but shifted to a higher molecular weight form over time upon induction of differentiation. All DNA-protein complexes were specific because they could be competed by a specific oligonucleotide whose sequence was derived from the E2F-binding site of the cyclin D1 promoter but not by an oligonucleotide of the mutated E2F-binding site.

### Example 3: Interference of pRB-E2F interaction

The shift of DNA-protein complexes to higher molecular weight forms during adipocyte differentiation suggests that the lower molecular weight form is a free, active E2F and the higher form is an inactive E2F in a larger complex, possibly with retinoblastoma protein pRB (Helin, K., 1998, Curr Opin Genet Dev 8(1): 28-35; Nevins, J. R., 1998, Cell Growth Differ 9(8): 585-93). Therefore, the potential of reactivating E2F in adipocytes, and thereby for modulating PAI-1 expression, by disrupting a possible pRB-E2F complex was addressed. Physiologically, pRB can be dissociated from E2F through hyperphosphorylation of pRB mediated by cdk 4/6, which is under the negative regulation of various inhibitory molecules of small molecular weight, such as p16^{INK} and p19^{ARF}. However, reactivation of E2F in adipocytes has not been described and this Example illustrates the invention by demonstrating physical competition of the pRB-E2F interaction.

The competition was examined by EMSA (see example 2) using an excess of a specific peptide, the sequence of which was derived from amino acids 402-419 of E2F1 corresponding to the pRB-binding region of E2F1 (Helin, K., et al., 1992, Cell 70(2): 337-50) or a control peptide with the same amino acid composition as the specific peptide but with a randomly shuffled sequence. In EMSA using radioactive E2pro oligonucleotide and nuclear extracts from day 8 NIH3T3-L1 cells, a specific interfering peptide was added to the binding reaction at increased concentration. A shift from DNA-protein complexes of higher molecular weight forms to complexes of lower molecular weight forms could be observed. The control peptide showed no effect. These results suggest the feasibility of using a specific peptide to interfere with the interaction between pRB and E2F.

The specific peptide was modified to improve cell penetration by linking to the amino terminal end of the interfering peptide a specific region of the HIV tat protein of 18 amino acids in length, which renders conjugated peptides cell-penetrable (Schwarze, S. R., et al., 1999, Science 285(5433): 1569-72). The effect of this elongated interfering peptide on E2F was first assessed *in vitro* as above. DNA-protein complexes on the E2F-binding site of the adenovirus E2 promoter shifted to lower molecular weight forms when increasing concentrations of the specific cell penetrating peptide were added to binding mixtures, while a control cell-penetrating peptide showed no effect. The result indicates that the addition of 11 amino acids to the amino terminal of the interfering peptide does not affect its ability to disrupt E2F-pRB complex. We then tested whether the peptide could interfere with the E2F-pRB interaction *in vivo.* For this, adipocytic cells (8 days after induction) were first treated with the cell-penetrating interfering peptide at different concentrations for 16 h and nuclear extracts were prepared for analysis by EMSA. The cell-penetrating interfering peptide but not the control peptide shifted DNA-protein complexes to lower molecular weight forms. These results indicate that the specific cell-penetrating peptide indeed penetrates cells and converts E2F-pRB complex to free E2F.

### Example 4: Effect of cell-penetrating peptides on PAI-1 mRNA levels

As mentioned in Example 1, the results show that E2F is post-transcriptionally regulated in adipocytes. Most likely E2F is in an inactive state complexed with the pocket-binding protein pRB (see Example 3). In order to activate E2F, the penetrating peptide system (Lindgren, M. et al., 2000, Trends Pharmacol Sci. 21:99-103) has been used to dissociate pRB from E2F. Therefore a cell-permeable peptide is constructed in which a peptide of interest is linked at the carboxyl terminal of a cell-penetrating peptide. The pRB-binding region in the E2F1 located in its carboxyl terminal region has been shown to be 18-amino acid long and conserved among mammalian species. The peptide of this sequence has been connected to the synthetic penetrating peptide, (KLAL)₄LA (SEQ ID NO: 4; Oehlke, J. et al., 1998, Biochim Biophys Acta. 1414:127-139). In this example, reactivation of E2F by disrupting E2F-pRB complex in adipocytes by a penetrating peptide was examined which could lead to downregulation of the PAI-1 gene expression. Cell-penetrating peptide was prepared by connecting a 18 aa-peptide corresponding to the pRB-binding region of E2F1 (Helin, K., et al., 1992, Cell 70(2): 337-50) with the cell-penetrating region of HIV tat protein (aa 47-57) (Schwarze, S. R., et al., 1999, Science 285(5433): 1569-72). As a control, a peptide of the same length and amino acid composition but with a shuffled sequence was connected. The penetrating peptide was added to culture medium, and cells were incubated for 16 h and then induced with or without 10 µg/ml insulin for 2 h. E2F activity and levels of PAI-1 mRNA were measured by electromobility shift assays (see Example 2) and Northern blot hybridization (see Example 1), respectively. It was shown that the penetrating specific peptide reduced both basal and insulin-induced PAI-1 mRNA levels, the latter being more potently affected and the effect reaching optimal at 30 µM. The control penetrating peptide had no effect.

### Example 5: Inhibition of PI3K pathway, enhanced PAI-1 gene expression at basal level or after induction with insulin, TNFα or glucose

Insulin-induced signaling pathways involving Shc and P13K can be independently inhibited. P13K activity can be specifically inhibited by wortmannin (Kanai, F. et al., 1993, Biochem Biophys Res Commun. 195:762-768) and Shc-mediated activation of the AP1 can be inhibited by the MEK1 inhibitor PD98059 (Servant, M. J. et al., 1996, J Biol Chem. 271:16047-16052). In this example, adipocytes are treated by insulin in the presence or absence of these inhibitors and then PAI-1 expression is examined (for example, by Northern blot analysis; see Example 1). In the presence of P13K inhibitors, PAI-1 induction by insulin is enhanced while in the presence of MEK1 inhibitors PAI-1 induction by insulin is suppressed suggesting that insulin exerts both positive and negative signals to the PAI-1 promoter in adipocytes. Alternatively, glucose and TNFα can be examined as potential positive signals for the PAI-1 gene (as described in Chen, Y. Q. et al., 1998, J. Biol. Chem., 273, 8225-8231; or Samad, F., et al., 1996, J. Clin. Invest. 97: 37-46).

### Example 6: Activation/inactivation of αvβ3 integrin, insulin-induced activation of P13K

αvβ3 integrins can be activated or inhibited using the activating monoclonal antibody LM609 (Charo, I. F. et al., 1990, J Cell Biol. 111:2795-2800) or the snake venom echistatin A (Fisher, J. E. et al., 1993, Endocrinology. 132:1411-1413), respectively. Adipocyte cells plated on vitronectin-coated dishes are treated by insulin in the presence or absence of these reagents. The effects of these reagents on P13K-mediated glucose uptake are examined in order to establish the involvement of αvβ3 in insulin-mediated signaling. Glucose uptake has been measured using {³H}-labeled 2-deoxyglucose (Standaert, M. L. et al., 1997, J Biol Chem. 272:30075-30082). The effect of these reagents on insulin-induced tyrosine phosphorylation of IRS-1 and activation of P13K is assessed. LM606 enhances and echistatin A suppresses insulin induced glucose uptake to show that PAI-1 can also be the cause of insulin resistance through modulating αvβ3 integrin function.

### Example 7: Association of NIDDM with obesity: Insulin-induced glucose uptake enhanced in PAI-1 knockout obese mice

PAI-1 knockout mice (Carmeliet, P. et al., 1995, Ann N Y Acad Sci. 748:367-381; discussion 381-382) or normal mice have been crossed with obese-hyperglycaemic mice (ob/ob) and the glucose uptake ability of adipocytes of (PAI-1+/+, ob/ob) and (PAI-1-/-, ob/ob) mice has examined in vivo and in vitro. Insulin induced glucose uptake is higher in PAI-1-/-, ob/ob mice than in PAI-1 +/+,ob/ob mice, demonstrating that PAI-1 plays a causative role for insulin resistance in NIDDM.

All references referred to herein, as well as priority application GB 0130007.8 filed December 14, 2001, are hereby referred to individually.

## Claims

1. A method of screening for agents effective against diabetes, said method comprising:
(a) assaying for PAI-1 activity in the presence of a candidate agent; and
(b) correlating a decrease in PAI-1 activity relative to when said candidate agent is absent with the presence of a potential agent effective against diabetes.

2. The method of claim 1, wherein said PAI-1 activity is PAI-1 expression.

3. The method of claim 1, wherein step (a) comprises detecting an increase in E2F activity.

4. The method of claim 3, wherein said increase in E2F activity is mediated by disrupting the interaction between E2F and pRB.

5. The method of claim 4, wherein said interaction between E2F and pRB is disrupted using a peptide.

6. The method of claim 1, wherein step (a) comprises detecting an increase in αvβ3 activity.

7. The method of claim 6, comprising detecting the binding of vitronectin to αvβ3.

8. A method of screening for agents effective against diabetes, said method comprising:
(a) assaying for E2F activity in the presence of a candidate agent; and
(b) correlating an increase in E2F activity relative to when said candidate agent is absent with the presence of a potential agent effective against diabetes.

9. The method of claim 8, wherein said E2F activity is detected by monitoring PAI-1 expression.

10. The method of claim 8, wherein step (a) comprises detecting a decrease in PAI-1 activity.

11. The method of claim 8, comprising assaying for an association between E2F and pRB in the presence of a candidate agent, wherein a loss in said association relative to when said candidate agent is absent is indicative of an increase in E2F activity.

12. The method of claim 11, wherein said candidate agent is a peptide or peptide mimetic.

13. Use of E2F or an active fragment thereof in the production of a medicament for the treatment or prophylaxis of diabetes.

## Patentansprüche

1. Verfahren zum Screenen auf Mittel, die gegen Diabetes wirksam sind, wobei das Verfahren umfasst:
(a) Test auf PAI-1 Aktivität in Gegenwart eines Kandidatenmittels und
(b) Korrelation einer Abnahme der PAI-1 Aktivität in Gegenwart eines potentiellen gegen Diabetes wirksamen Mittels relativ dazu, wenn das Kandidatenmittel abwesend ist.

2. Verfahren nach Anspruch 1, worin die PAI-1 Aktivität die PAI-1 Expression ist.

3. Verfahren nach Anspruch 1, worin der Schritt (a) die Detektion einer Zunahme der E2F Aktivität umfasst.

4. Verfahren nach Anspruch 3, worin die Zunahme der E2F Aktivität durch die Zerstörung der Wechselwirkung zwischen E2F und pRB vermittelt wird.

5. Verfahren nach Anspruch 4, worin die Wechselwirkung zwischen E2F und pRB mittels eines Peptids zerstört wird.

6. Verfahren nach Anspruch 1, worin der Schritt (a) die Detektion einer Zunahme der αvβ3 Aktivität umfasst.

7. Verfahren nach Anspruch 6, das die Detektion der Bindung von Vitronektin an αvβ3 umfasst.

8. Verfahren zum Screenen auf Mittel, die gegen Diabetes wirksam sind, wobei das Verfahren umfasst:
(a) Test auf E2F Aktivität in Gegenwart eines Kandidatenmittels, und
(b) Korrelation einer Zunahme der E2F Aktivität in Gegenwart eines potentiellen gegen Diabetes wirksamen Mittels relativ dazu, wenn das Kandidatenmittel abwesend ist.

9. Verfahren nach Anspruch 8, worin die E2F Aktivität durch die Verfolgung der PAI-1 Expression detektiert wird.

10. Verfahren nach Anspruch 8, worin der Schritt (a) die Detektion einer Verringerung der PAI-1 Aktivität umfasst.

11. Verfahren nach Anspruch 8, das einen Test auf eine Assoziation zwischen E2F und pRB in Gegenart eines Kandidatenmittels umfasst, worin der Verlust der Assoziation relativ dazu, wenn das Kandidatenmittel abwesend ist, eine Erhöhung der E2F Aktivität anzeigt.

12. Verfahren nach Anspruch 11, worin das Kandidatenmittel ein Peptid oder Peptidmimetikum ist.

13. Verwendung von E2F oder eines aktiven Fragments hiervon zur Herstellung eines Arzneimittels zur Behandlung oder Prophylaxe von Diabetes.

## Revendications

1. Procédé de sélection d'agents efficaces contre le diabète, comprenant les étapes consistant à :
(a) tester l'activité de PAI-1 en présence d'un agent candidat ; et
(b) établir une corrélation entre une diminution de l'activité de PAI-1 associée à l'absence dudit agent candidat et la présence d'un agent potentiel efficace contre le diabète.

2. Procédé selon la revendication 1, dans lequel ladite activité de PAI-1 est l'expression de PAI-1.

3. Procédé selon la revendication 1, dans lequel l'étape (a) comprend la détection d'une augmentation de l'activité de E2F.

4. Procédé selon la revendication 3, dans lequel ladite augmentation de l'activité de E2F passe par la dissociation de l'interaction entre E2F et pRB.

5. Procédé selon la revendication 4, dans lequel ladite interaction entre E2F et pRB est dissociée par utilisation d'un peptide.

6. Procédé selon la revendication 1, dans lequel l'étape (a) comprend la détection d'une augmentation de l'activité de αvβ3.

7. Procédé selon la revendication 6, comprenant la détection de la liaison de la vitronectine à αvβ3.

8. Procédé de sélection d'agents efficaces contre le diabète, comprenant les étapes consistant à :
(a) tester l'activité de E2F en présence d'un agent candidat ; et
(b) établir une corrélation entre une augmentation de l'activité de E2F associée à l'absence dudit agent candidat et la présence d'un agent potentiel efficace contre le diabète.

9. Procédé selon la revendication 8, dans lequel ladite activité de E2F est détectée par le contrôle de l'expression de PAI-1.

10. Procédé selon la revendication 8, dans lequel l'étape (a) comprend la détection d'une baisse de l'activité de PAI-1.

11. Procédé selon la revendication 8, comprenant les étapes consistant à tester une association entre E2F et pRB en présence d'un agent candidat, dans lequel une perte de ladite association associée à l'absence dudit agent candidat indique une augmentation de l'activité de E2F.

12. Procédé selon la revendication 11, dans lequel ledit agent candidat est un peptide ou un peptide mimétique.

13. Utilisation de E2F ou de l'un de ses fragments actifs dans la fabrication d'un médicament pour le traitement ou la prophylaxie du diabète.


### SEQUENCE LISTING

<110> Novartis Forschungsstiftung Zweigniederlassung Friedrich Miescher Institute for Biomedical Research
<120> Screening Method for agents useful in treating diabetes
<130> 1-32277A/FMI
<140> GB-0130007.8
   <141> 2001-12-14
<160> 4
<170> PatentIn Ver. 2.1
<210> 1
   <211> 42
   <212> DNA
   <213> Artificial sequence
<220>
<223> Description of Artificial Sequence:oligonucleotide
<400> 1
   atcagttttc gcgcttaaat ttgagaaagg gcgcgaaact ag 42
<210> 2
   <211> 33
   <212> DNA
   <213> Artificial Sequence
<220>
<223> Description of Artificial Sequence:oligonucleotide
<400> 2
   aattcgctgc tcccggcgtt tggcgcccgc gcc 33
<210> 3
   <211> 24
   <212> DNA
   <213> Artificial Sequence
<220>
   <223> Description of Artificial Sequence:oligonucleotide
<400> 3
   aattcgctgc tcccgtcgtt aggtgtccgc gcc 33
<210> 4
   <211> 18
   <212> PRT
   <213> Artificial Sequence
<220>
   <223> Description of Artificial Sequence:peptide
<400> 4
   Lys Leu Ala Leu Lys Leu Ala Leu Lys Leu Ala Leu Lys Leu Ala Leu
    1               5                  10                  15
   Leu Ala

### SEQUENCE LISTING

<110> Novartis Forschungsstiftung Zweigniederlassung Friedrich Miescher Institute for Biomedical Research
<120> Screening Method for agents useful in treating diabetes
<130> 1-32277A/FMI
<140> GB-0130007.8
   <141> 2001-12-14
<160> 4
<170> PatentIn Ver. 2.1
<210> 1
   <211> 42
   <212> DNA
   <213> Artificial sequence
<220>
   <223> Description of Artificial Sequence:oligonucleotide
<400> 1
   atcagttttc gcgcttaaat ttgagaaagg gcgcgaaact ag 42
<210> 2
   <211> 33
   <212> DNA
   <213> Artificial Sequence
<220>
<223> Description of Artificial Sequence:oligonucleotide
<400> 2
   aattcgctgc tcccggcgtt tggcgcccgc gcc 33
<210> 3
   <211> 24
   <212> DNA
   <213> Artificial Sequence
<220>
   <223> Description of Artificial Sequence:oligonucleotide
<400> 3
   aattcgctgc tcccgtcgtt aggtgtccgc gcc 33
<210> 4
   <211> 18
   <212> PRT
   <213> Artificial Sequence
<220>
   <223> Description of Artificial Sequence:peptide
<400> 4
   Lys Leu Ala Leu Lys Leu Ala Leu Lys Leu Ala Leu Lys Leu Ala Leu
    1               5                  10                  15
   Leu Ala

### SEQUENCE LISTING

<110> Novartis Forschungsstiftung Zweigniederlassung Friedrich Miescher Institute for Biomedical Research
<120> Screening Method for agents useful in treating diabetes
<130> 1-32277A/FMI
<140> GB-0130007.8
   <141> 2001-12-14
<160> 4
<170> PatentIn Ver. 2.1
<210> 1
   <211> 42
   <212> DNA
   <213> Artificial sequence
<220>
<223> Description of Artificial Sequence:oligonucleotide
<400> 1
   atcagttttc gcgcttaaat ttgagaaagg gcgcgaaact ag 42
<210> 2
   <211> 33
   <212> DNA
   <213> Artificial Sequence
<220>
<223> Description of Artificial Sequence:oligonucleotide
<400> 2
   aattcgctgc tcccggcgtt tggcgcccgc gcc 33
<210> 3
   <211> 24
   <212> DNA
   <213> Artificial Sequence
<220>
   <223> Description of Artificial Sequence:oligonucleotide
<400> 3
   aattcgctgc tcccgtcgtt aggtgtccgc gcc 33
<210> 4
   <211> 18
   <212> PRT
   <213> Artificial Sequence
<220>
   <223> Description of Artificial Sequence:peptide
<400> 4
   Lys Leu Ala Leu Lys Leu Ala Leu Lys Leu Ala Leu Lys Leu Ala Leu
    1               5                  10                  15
   Leu Ala